# EUROPEAN PATENT APPLICATION

(11) **EP 3 238 894 A1**
(43) Date of publication of application: **01.11.2017**
(21) Application number: 15872859.2
(22) Date of filing: 16.12.2015
(51) Int. Cl.: B25J 19/06, B25J 18/02

(54) **ROBOT SYSTEM AND ROBOT DEVICE**

(30) Priority: 25.12.2014 JP 2014263625
(71) Applicant: Life Robotics Inc., Koto-ku Tokyo 135-0047 (JP)
(72) Inventor: TAKASE, Sosuke, Tokyo 135-0047 (JP)
(74) Representative: Herrero & Asociados, S.L.
(86) International application number: PCT/JP2015/085233
(87) International publication number: WO 2016/104290

(57) **Abstract**

This invention provides an efficient robot layout in which there is a high degree of freedom. The robot system includes a work bench on which a work object is placed, and a robot device. The robot device includes: a base (1); and an arm section (2) that has torsional rotatability around a first axis approximately along a center line of the base, bending rotatability around a second axis that is orthogonal to the first axis, and linear extensibility and retractability along a third axis that is orthogonal to the second axis. Work is performed in collaboration with a worker on the work object by means of an end effector (3) that is provided at a tip of the arm section. The base is disposed at a position at which a moveable region (MR) of the arm section accompanying rotation around the first axis and the second axis and linear extension and retraction along the third axis at least partially overlaps with a work region (WAM) of the worker.

## Description

### FIELD

Embodiments described herein relate generally to a robot system and a robot device.

### BACKGROUND

Recent years have seen an increasing number of environments in which a robot and a user are present in the same space. In addition to robots for nursing care that naturally carry out work in the same space as a user, it is considered that from now on there will be an increasing number of situations in which industrial robots perform work side-by-side with workers. Many of such robots are equipped with a vertically articulated arm mechanism. A vertically articulated arm mechanism is required to have three degrees of freedom (x, y, z) in relation to position and three degrees of freedom (φ, θ, ψ) in relation to posture, and generally this is realized by revolute joints J1, J2 and J3 which are called root three axes, and revolute joints J4, J5 and J6 which are called wrist three axes.

Because translational movement of ends requires rotation of the joints J1, J2, J3 in operative association with each other, in particular the motion of the joint J2 that is called an "elbow section" is extremely large and the motion thereof cannot be predicted, and a situation in which the motion of the elbow section becomes extremely fast in the vicinity of a singular point, in particular, is unavoidable.

Therefore, in order to avoid contact between a robot and a worker or with another structure in the area around the robot, it has been essential to provide extra space or to install a safety fence around the robot, and such kind of robot layout has been extremely inefficient.

### BRIEF DESCRIPTION OF THE INVENTION

A purpose of the present invention is to provide a robot layout which has a high degree of freedom and which is efficient.

A robot system according to the present embodiment includes a work bench on which a work piece is placed, and a robot device. The robot device includes: a base; and an arm section having torsional rotatability around a first axis approximately along a center line of the base, bending rotatability around a second axis that is orthogonal to the first axis, and linear extensibility and retractability along a third axis that is orthogonal to the second axis. Work is performed in collaboration with a worker with respect to the work piece by means of an end effector that is mounted at a tip of the arm section. The base is disposed so that a moveable region of the arm section accompanying rotation around the first axis and the second axis and linear extension and retraction along the third axis overlaps a working region of the worker.

### BRIEF DESCRIPTION OF THE VIEWS OF THE DRAWINGS

FIG. 1 is an external perspective view of an arm mechanism of a robot device according to an embodiment;
FIG. 2 is a perspective view illustrating an internal structure of the robot arm mechanism in FIG. 1;
FIG. 3 is a view illustrating an internal structure of the robot arm mechanism in FIG. 1, which is a cross section view;
FIG. 4 is a view illustrating the robot arm mechanism in FIG. 1 by representation with graphic symbols;
FIGS. 5A and 5B are views illustrating extension lengths of a linear extension and retraction joint of the robot arm mechanism shown in FIG. 1;
FIGS. 6A to 6C are views illustrating a moveable region of an arm section of the robot arm mechanism shown in FIG. 1;
FIGS. 7A and 7B are views illustrating a layout example in which a base of the robot arm mechanism shown in FIG. 1 is disposed adjacent to a worker;
FIGS. 8A and 8B are views illustrating a layout example in which the base of the robot arm mechanism shown in FIG. 1 is disposed at an angle of 90° to the right of a worker;
FIGS. 9A and 9B are views illustrating a layout example in which the base of the robot arm mechanism shown in FIG. 1 is disposed facing a worker;
FIG. 10 is a view illustrating a layout example in which the base of the robot arm mechanism shown in FIG. 1 is disposed in front of a worker;
FIG. 11 is a view illustrating a situation in which a working region of the robot arm mechanism shown in FIG. 1 can overlap with a working region of a worker;
FIGS. 12A and 12B are views illustrating layout examples for the base of the robot arm mechanism shown in FIG. 1 with respect to a plurality of workers; and
FIGS. 13A and 13B are views illustrating layout examples for the bases of two of the robot arm mechanisms shown in FIG. 1 with respect to a worker.

### DETAILED DESCRIPTION

Hereinafter, a robot system and a robot device according to the present embodiment are described with reference to the accompanying drawings. The robot system is included a work bench on which work pieces are placed, and a robot device for performing work on the work pieces in collaboration with a worker. The robot device is constituted by a robot arm mechanism, and a main body section that supplies power to the robot arm mechanism and also controls operations of the robot arm mechanism. The robot arm mechanism is constituted by a base and an arm section that is supported by the base. An end effector such as a hand that performs work is mounted at the tip of the arm section. First, the robot arm mechanism will be described.

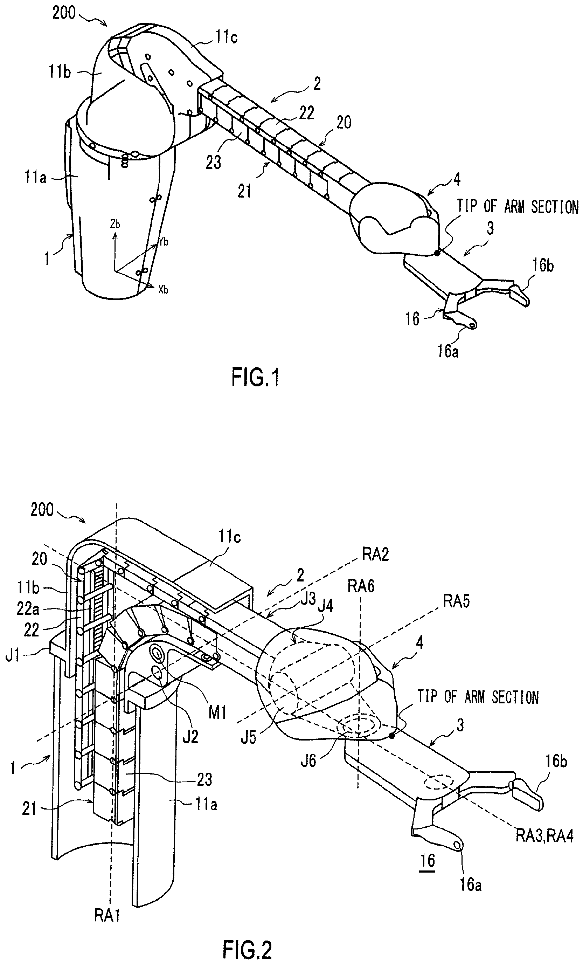

A robot arm mechanism 200 illustrated in FIG. 1 includes a substantially cylindrical base 1 and an arm section 2 that is supported by the base 1. An end effector 3 is attached to the tip of the robot arm section 2. In FIG. 1, a hand section capable of holding an object is shown as the end effector 3. The end effector 3 is not limited to the hand section, and may be another tool, a camera, or a display. At the tip of the robot arm section 2, an adapter which can be replaced with any type of the end effector 3 may be mounted.

The robot arm section 2 includes a plurality (herein, six) of joints J1, J2, J3, J4, J5 and J6. The joints J1, J2, J3, J4, J5 and J6 are arranged in order from the base 1. Generally, a first axis RA1, a second axis RA2 and a third axis RA3 are called root three axes, and a fourth axis RA4, a fifth axis RA5 and a sixth axis RA6 are called wrist three axes. A portion that equips the joints J4, J5 and J6 that provide the wrist three axes is called a wrist section 4, and the physical tip of the wrist section 4 is defined as the tip of the arm section. At least one the joints J1, J2 and J3 providing the root three axes is a linear motion joint. Herein, the third joint J3 is formed as a linear motion joint, in particular, as a joint with a relatively long extension and retraction distance. The first joint J1 is a torsion joint that has on the first axis of rotation RA1 and which is held, for example, perpendicularly to a base surface. The second joint J2 is a bending joint that has the second axis of rotation RA2 perpendicular to the first axis of rotation RA1. The third joint J3 linearly extends or retracts along the third axis (axis of movement) RA3 perpendicular to the second axis of rotation RA2. The fourth joint J4 is a torsion joint that rotates on the fourth axis of rotation RA4 which matches the third axis of movement RA3. The fifth joint J5 is a bending joint that has the fifth axis of rotation RA5 orthogonal to the fourth axis of rotation RA4. The sixth joint J6 is a bending joint that has the sixth axis of rotation RA6 orthogonal to the fourth axis of rotation RA4 and perpendicular to the fifth axis of rotation RA5.

The arm section 2 turns together with the hand section 3 in accordance with torsional rotation of the first joint J1. The arm section 2 rotates upward and downward on the second axis of rotation RA2 of the second joint J2 together with the hand section 3 in accordance with bending rotation of the second joint J2. An arm support body (first support body) 11a forming the base 1 has a cylindrical hollow structure formed around the axis of rotation RA1 of the first joint J1. The first joint J1 is mounted on a fixed base (not shown). When the first joint J1 rotates, the first support body 11a axially rotates in accordance with the turn of the arm section 2. The first support body 11a may be fixed on a ground plane. In this case, the arm section 2 turns independently of the first support body 11a. A second support body 11b is connected to an upper part of the first support body 11a.

The second support body 11b has a hollow structure continuous to the first support body 11a. One end of the second support body 11b is attached to a rotating section of the first joint J1. The other end of the second support body 11b is opened, and a third support body 11c is set rotatably on the axis of rotation RA2 of the second joint J2. The third support body 11c has a scaly hollow structure communicating with the first support body 11a and the second support body. In accordance with the bending rotation of the second joint J2, a rear part of the third support body 11c is accommodated in or sent out from the second support body 11b. The rear part of the third joint J3, which constitutes a linear motion joint of the arm section 2, is housed inside the continuous hollow structure of the first support body 11a and the second support body 11b by retraction thereof.

The first joint J1 includes an annular fixed section and a rotating section, and is fixed to a base mount (not shown) at the fixed section. The first support body 11a and the second support body 11b are attached to the rotating section. When the first joint J1 rotates, the first support body 11a, the second support body 11b and the third support body 11c turn around the first axis of rotation RA1 together with the arm section 2 and the hand section 3.

The third support body 11c is set rotatably, at the lower part of its rear end, on the axis of rotation RA2 with respect to a lower side of an open end of the second support body 11b. In this way, the second joint J2 serving as a bending joint that rotates on the axis of rotation RA2 is formed. When the second joint J2 rotates, the arm section 2 rotates vertically, i.e., rotates upward and downward, on the axis of rotation RA2 of the second joint J2 together with the hand section 3.

As described above, the third joint J3 serving as a joint section constitutes a main constituent of the arm section 2. The hand section 3 described above is mounted at the tip of the arm section 2. Rotation, bending, and extension and retraction of the first to sixth joints J1-J6 enable positioning of a two-fingered hand 16 of the hand section 3 at a given position and posture. In particular, the length of the linear extension and retraction distance of the third joint J3 enables the hand section 3 to act on an object in a wide range from a position close to the base 1 to a position far from the base 1.

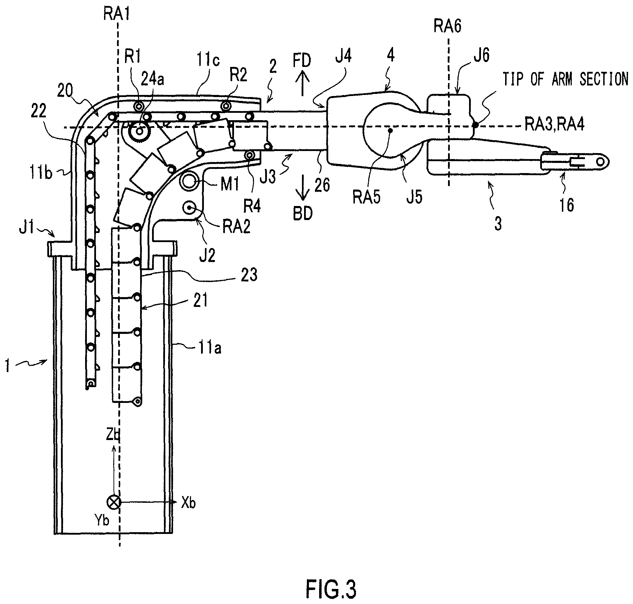

The third joint J3 is characterized by the length of the linear extension and retraction distance realized by a linear extension and retraction arm mechanism constituting the third joint J3. The length of the linear extension and retraction distance is achieved by the structure shown in FIG. 2 and FIG. 3. The linear extension and retraction arm mechanism includes a first connection piece string 21 and a second connection piece string 20. In a reference pose where the arm section 2 is horizontal, the first connection piece string 21 is located below the second connection piece string 20, and the second connection piece string 20 is located above the first connection piece string 21.

The first connection piece string 21 includes a plurality of first connection pieces 23 having the U-shaped cross section and concatenated to form a string by pins at their bottom parts. The first connection piece string 21 is bendable in its bottom direction BD but conversely not bendable in its upper side direction FD due to the shape of the cross section of the first connection piece 23 and the positions of connection by the pins. The second connection piece string 20 has a substantially flat plate shape with a width substantially equivalent to that of the first connection piece 23, and includes a plurality of second connection pieces 22 concatenated to form a string by pins in a bendable state in the upper side direction. The tip of the first connection piece string 21 is joined to the tip of the second connection piece string 20 by a head piece 26. The head piece 26 has a shape in which the first connection piece 23 and the second connection piece 22 are integrated. When the second connection piece string 20 is sent out from the third support body 11c together with the first connection piece string 21, the second connection piece string 20 is stacked on the first connection piece string 21. The stacked state is maintained. When the stacked state between the first connection piece string 21 and the second connection piece string 20 is maintained, bending of the first connection piece string 21 and the second connection piece string 20 is restricted, whereby the first connection piece string 21 and the second connection piece string 20 constitute a columnar body having a certain rigidity. When the first connection piece string 21 and the second connection piece string 20 are separated from each other, the bending restriction is released, and the first connection piece string 21 and the second connection piece string 20 each revert to a bendable state. The first connection piece string 21 and the second connection piece string 20 are stacked in the third support body 11c to be sent out. The first connection piece string 21 and the second connection piece string 20 are separated from each other at the rear of the third support body 11c, and each returns a bendable state. The first connection piece string 21 and the second connection piece string 20 are bent individually to be accommodated as separate bodies on the inner side of the first support body 11a.

As shown in FIG. 2, a linear gear 22a is formed on the underside of each of the second connection pieces 22. The linear gears 22a are connected to form a continuous linear gear when the second connection pieces 22 are arranged in a linear shape. As shown in FIG. 3, the second connection piece 22 is sandwiched between a roller R1 and a drive gear 24a inside the third support body 11c. The linear gear 22a is engaged with the drive gear 24a. When the drive gear 24a is rotated forward by a motor M1, the second connection piece string 20 is sent out from the third support body 11c together with the first connection piece string 21. At that time, the first connection piece string 21 and the second connection piece string 20 are sandwiched between a pair of upper and lower rollers R2 and R4 arranged in the vicinity of the opening of the third support body 11c, and are sent out linearly along the third axis of movement RA3 in a state in which the first connection piece string 21 and the second connection piece string 20 are pressed and stacked to each other. When the drive gear 24a is rotated backward by the motor M1, the stacked state between the second connection piece string 20 and the first connection piece string 21 is released and the second connection piece string 20 and first connection piece string 21 are separated from each other at the rear side of the upper and lower rollers R2 and R4 inside the third support body 11c. The separated second connection piece string 20 and first connection piece string 21 each enter the bendable state, and are guided by guide rails arranged inside the second support body 11b and the third support body 11c to be bent in a direction along the first axis of rotation RA1 and housed inside the first support body 11a.

The hand section 3 is mounted at the tip of the arm section 2 as shown in FIG. 1. The hand section 3 is moved to any position by the first joint J1, the second joint J2 and the third joint J3, and placed in any posture by the fourth joint J4, the fifth joint J5 and the sixth joint J6. The hand section 3 includes two fingers 16a and 16b configured to be opened and closed. The fourth joint J4 is a torsion joint having the axis of rotation RA4 which typically matches a center axis of the arm section 2 along the extension and retraction direction of the arm section 2, that is, the axis of movement RA3 of the third joint J3. When the fourth joint J4 rotates, the hand section 3 rotates on the axis of rotation RA4 from the fourth joint J4 to the tip thereof. An end coordinate system Eh is defined that takes an end reference point of the end effector 3, in this case, a center point between the two fingers 16a and 16b of the hand section 3, as an origin ∑h0.

The fifth joint J5 is a bending joint having an axis of rotation RA5 orthogonal to the axis of movement RA4 of the fourth joint J4. When the fifth joint rotates, the hand section 3 pivots up and down from the fifth joint J5 to the tip of the arm section 2 together with the hand 16. The sixth joint J6 is a bending joint having the axis of rotation RA6 orthogonal to the axis of rotation RA4 of the fourth joint J4 and perpendicular to the axis of rotation RA5 of the fifth joint J5. When the sixth joint J6 rotates, the hand 16 turns left and right.

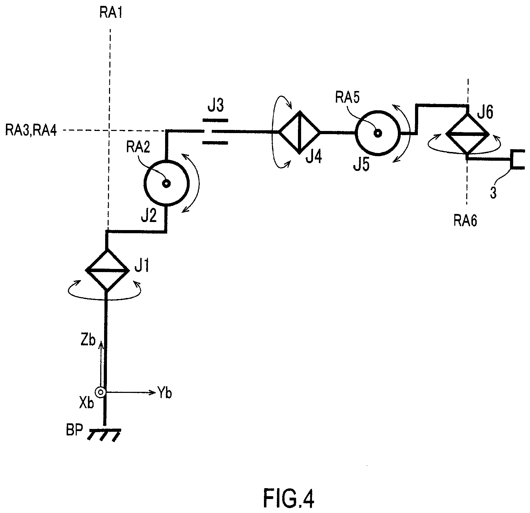

The robot arm mechanism shown in FIG. 1 is illustrated by representation with graphic symbols in FIG. 4. The robot arm mechanism realizes three positional degrees of freedom and three postural degrees of freedom by means of the first joint J1, the second joint J2 and the third joint J3 constituting the root three axes, and the fourth joint J4, the fifth joint J5 and the sixth joint J6 constituting the wrist three axes. The first joint J1 is arranged between the first support body 11a and the second support body 11b, and is configured as a torsion joint that rotates on the axis of rotation RA1. The axis of rotation RA1 is arranged perpendicular to a base plate BP of the base mount on which the fixed section of the first joint J1 is disposed. A reference coordinate system (Xb, Yb, Zb) having three orthogonal axes is defined that takes any point on the axis of rotation RA1 as the origin. A Zb axis that is parallel to the axis of rotation RA1 and an Xb that is parallel to the axis of rotation RA2 are defined. The reference coordinate system is defined as a rotating coordinate system that rotates together with rotation of the first joint J1.

The second joint J2 is configured as a bending joint that rotates on the axis of rotation RA2. The axis of rotation RA2 of the second joint J2 is arranged parallel to the Xb axis on a spatial coordinate system. The axis of rotation RA2 of the second joint J2 is arranged in a perpendicular direction relative to the axis of rotation RA1 of the first joint J1. In addition, relative to the first joint J1, the second joint J2 is offset in two directions, namely, the direction of the first axis of rotation RA1 (Zb-axis direction), and the Yb-axis direction that is perpendicular to the first axis of rotation RA1. An inter-joint distance (also referred to as "distance between joint centers", "link length" or "offset distance") relating to the Zb-axis direction between the first joint J1 and the second joint J2 is given by d1, and an inter-joint distance relating to the Yb-axis direction between the first joint J1 and the second joint J2 is given by L1. Note that, the joint center of a revolute joint refers to the structural center on the plane of rotation, and the joint center of a linear motion joint refers to the structural center in a most retracted state.

The second support body 11b is attached to the first support body 11a so that the second joint J2 is offset in the aforementioned two directions relative to the first joint J1. A virtual arm rod portion (link portion) connecting the first joint J1 to the second joint J2 has a crank shape in which two hook-shaped bodies that each have a tip that is bent at a right angle are combined. The virtual arm rod portion is constituted by the first and second support bodies 11a and 11b which have a hollow structure.

The third joint J3 is configured as a linear motion joint that rotates on the axis of movement RA3. The axis of movement RA3 of the third joint J3 is arranged in a perpendicular direction relative to the axis of rotation RA2 of the second joint J2. When the arm section 2 is in a horizontal reference pose in which the rotation angle of the second joint J2 is zero degrees, that is, the upward/downward rotation angle of the arm section 2 is zero degrees, the axis of movement RA3 of the third joint J3 is arranged in a perpendicular direction to the axis of rotation RA1 of the first joint J1 together with the axis of rotation RA2 of the second joint J2. On the spatial coordinate system, the axis of movement RA3 of the third joint J3 is arranged parallel to the Yb axis that is perpendicular to the Xb axis and Zb axis. In addition, relative to the second joint J2, the third joint J3 is offset in two directions, namely, the direction of the axis of rotation RA2 thereof (Yb-axis direction), and the direction of the Zb axis that is orthogonal to the axis of movement RA3. An inter-joint distance (offset distance) relating to the Zb-axis direction between the second joint J2 and the third joint J3 is given by d2, and an inter-joint distance relating to the Yb-axis direction between the second joint J2 and the third joint J3 is given by L2. The third support body 11c is attached to the second support body 11b so that the third joint J3 is offset in the aforementioned two directions relative to the second joint J2. A virtual arm rod portion (link portion) connecting the second joint J2 to the third joint J3 has a hook-shaped body in which the tip is bent at a right angle. The virtual arm rod portion is constituted by the second and third support bodies 11b and 11c.

The aforementioned inter-joint distance L2 relating to the Yb-axis direction between the first joint J1 and the second joint J2, and the inter-joint distance d2 relating to the Zb-axis direction between the second joint J2 and the third joint J3 are set to different values to each other.

The fourth joint J4 is configured as a torsion joint that rotates on the axis of rotation RA4. The axis of rotation RA4 of the fourth joint J4 is arranged so as to substantially match the axis of movement RA3 of the third joint J3. The fifth joint J5 is configured as a bending joint that rotates on the axis of rotation RA5. The axis of rotation RA5 of the fifth joint J5 is arranged so as to be substantially orthogonal to the axis of movement RA3 of the third joint J3 and the axis of rotation RA4 of the fourth joint J4. The sixth joint J6 is configured as a torsion joint that rotates on the axis of rotation RA6. The axis of rotation RA6 of the sixth joint J6 is arranged so as to be substantially orthogonal to the axis of rotation RA4 of the fourth joint J4 and the axis of rotation RA5 of the fifth joint J5. The sixth joint J6 is provided for turning the hand section 3 as an end effector, and the sixth joint J6 may be mounted as a bending joint in which the axis of rotation RA6 thereof is substantially orthogonal to the axis of rotation RA4 of the fourth joint J4 and the axis of rotation RA5 of the fifth joint J5.

By replacing one revolute joint among the root three axes of the plurality of joints J1 to J6 with the linear motion joint J6, causing the second joint J2 to be offset in two directions relative to the first joint J1, and causing the third joint J3 to be offset in two directions relative to the second joint J2 in this way, the structural elimination of a singular point posture is realized.

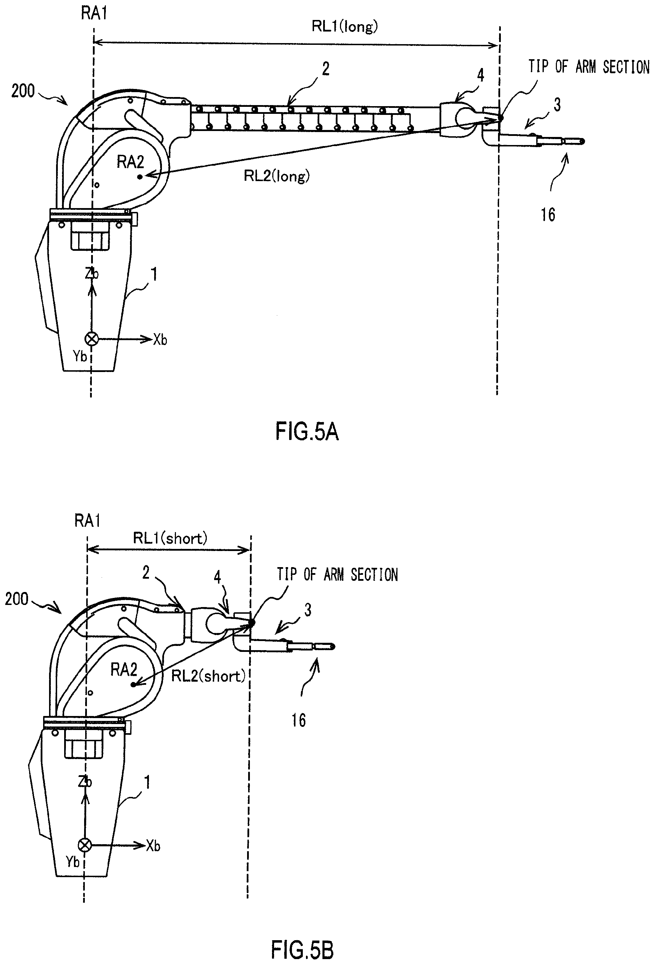

As shown in FIG. 5A, a length from the first axis RA1 to the tip of the arm section 2 when the linear motion joint J3 is extended to the maximum extent to place the arm section 2 in the longest state is referred to as "RL1 (long)", and a length from the second axis RA2 to the tip of the arm section 2 in the same state is referred to as "RL2 (long)". As shown in FIG. 5B, a length from the first axis RA1 to the tip of the arm section 2 when the linear motion joint J3 is retracted to the maximum extent to place the arm section 2 in the shortest state is referred to as "RL1 (short)", and a length from the second axis RA2 to the tip of the arm section 2 in the same state is referred to as "RL2 (short)".

In the present embodiment, a moveable region can be simplified because there is no elbow joint, and taking the arm lengths RL1 (long), RL2 (long), RL1 (short) and RL2 (short) achieved by the linear extensibility and retractability of the linear motion joint J3 as parameters, it is possible to efficiently design a robot layout that has a higher degree of freedom by means of a fan-shaped layout or the like that adopts these parameters as radii.

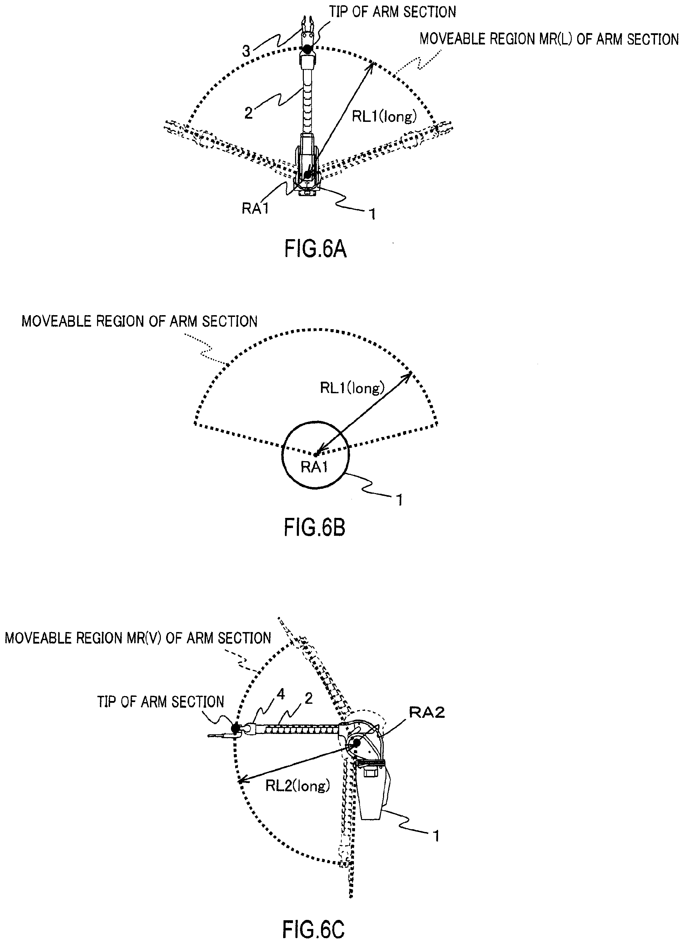

As shown in FIG. 6A and FIG. 6B, a region that the arm section 2 reaches by means of an operable rotation angle (turning angle) that can be implemented by the joints J1 and J3 and the extension distance of the arm mechanism 200 is referred to as a "moveable region MR(L)" of the arm section 2 in relation to the horizontal direction. In the arm mechanism 200, the moveable region MR(L) of the arm section 2 in relation to the horizontal direction is characterized by being definable as a substantially fan-shaped region for which the operating angle of the first joint J1 is taken as a central angle and the length RL1 (long) from the first axis RA1 to the tip of the arm section 2 when the linear motion joint J3 is extended to the maximum extent to provide the arm section 2 in the longest state thereof is taken as a radius. According to the present embodiment, this characteristic is used to expand the degree of freedom and increase the efficiency of the layout of the overall robot system in which the base 1 of the robot device is laid out with respect to a work bench, a worker, a surrounding wall surface and the like while adjusting the radius or central angle of the substantially fan-shaped region.

Further, a similar situation applies in relation to the perpendicular direction (direction of upward/downward rotation around the axis of rotation RA2 by the second joint J2) in which, as shown in FIG. 6B, when the arm section 2 is in the longest state in which the linear motion joint J6 is extended to the maximum extent, if the second joint J2 is axially rotated to rotate the arm section 2 upward and downward, a substantially fan-shaped moveable region MR(V) is drawn the center of which is the second axis RA2 and which takes the length RL2 (long) from the second axis RA2 to the tip of the arm section 2 as a radius. A three-dimensional moveable region MR of the arm section 2 is the combined region of the aforementioned fan-shaped moveable region MR(L) in the horizontal direction and the moveable region MR(V) relating to the perpendicular direction, and exhibits lengthwise and breadthwise fan shapes formed by rotating the fan-shaped moveable region MR(L) upward and downward within the operating range of the second joint J2. The term "front of the base 1" refers to the direction of a center line of the moveable region MR.

In this case, the important point is that, in the linear extension and retraction arm mechanism of the robot device according to the present embodiment, as described above, because there is no elbow joint, and a linear extension and retraction joint is used in place of the elbow joint, there is a comparatively small danger since the arm section 2 including the joints substantially does not project to the outside of the substantially fan-shaped moveable region MR, and furthermore, because the arm section 2 moves in a linear manner from the end to the base 1, a worker or the like can predict with a high degree of predictability the motion of the arm section 2 based on the motion of the end, and thus the danger can be further reduced.

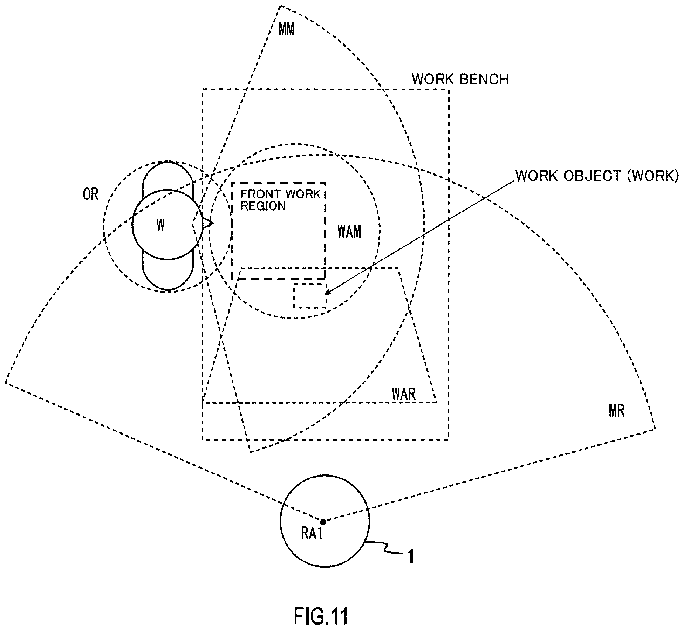

Accordingly, when laying out, in particular, the base 1 of the robot device in accordance with the work contents or surrounding environment, a layout can be realized that takes into consideration the aforementioned substantially fan-shaped moveable region MR, and spatial relationships such as interference or contact with human elements such as a worker, structural elements such as a tool shelf, and also facility elements such as a wall surface which is planned to have in place or present when performing the work, together with the operating range (operational turning angle) around the first axis RA1 of the first joint J1, the operating range (operational upward/downward rotation angle) around the second axis RA2 of the second joint J2, and the operating range (operational extension length) of the third joint J3. In particular, the fact that the degree of predictability regarding danger is high means that it is practically possible to lay out the base 1 so that the aforementioned moveable region MR of the substantially fan-shaped arm section 2 is arranged in the vicinity of a worker or the like, in other words, so that the moveable region MR of the arm section 2 interferes with an interference object or person, for example, a worker W, positioned at the periphery thereof, and by this means the moveable region MR of the arm section 2 or a working region WAR in which work is actually assigned to the robot device inside the moveable region MR can partially overlap with a reach region MM of the fingers of the worker or the like or partially overlap with a working region WAM (see FIG. 11) in which work is actually carried out inside the reach region MM, and thus the robot device can be caused to perform work in collaboration with a worker on the same work piece.

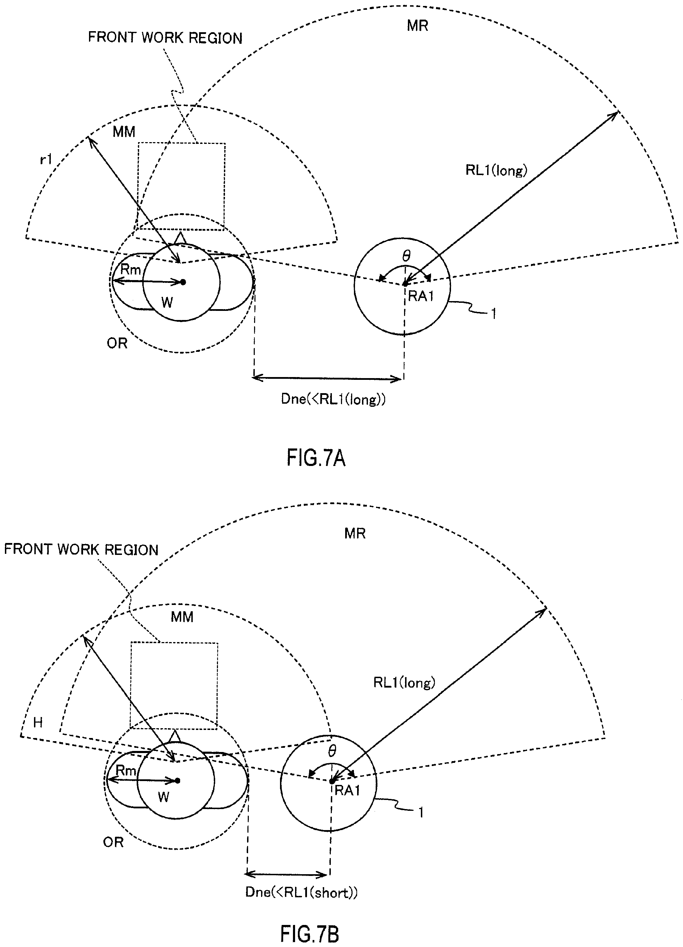

An example of a layout in which the base 1 of the robot device is arranged adjacent to a worker W is shown in FIG. 7A. As described above, the moveable region MR of the arm section 2 is a region that exhibits fan shapes in the lengthwise and breadthwise directions with respect to the radius RL1 (long) that takes an operable turning angle and an upward/downward rotation angle as fan center angles the centers of which are each the first axis RA1 and the second axis RA2 of the base 1 of the robot device. Note that, in the layout design, a reach region MM of the fingers that forms a substantially fan shape that takes an assumed length r1 from a shoulder of one arm to the fingers of the worker W in front of the worker W as a radius is set in advance. Further, a circular occupied region OR having a radius Rm that is in accordance with a standard physique or an assumed physique the center of which is the working position of the worker W is arranged around the worker W.

In addition, a front working region is arranged at the front of the worker W. The term "front working region" refers to a region which is in front of the worker W and which exhibits a comparatively high working efficiency by, for example, enabling collaborative work with both hands. Note that, the front working region should be distinguished from a working region that is described later. That is, the term "working region" refers to a region in which work is assigned to the worker W, that is, work on a work piece (work) within the working region is assigned to the worker W. In contrast, the term "front working region" is a region of a size of, for example, 60 cm x 60 cm that, ergonomically, is in accordance with an assumed physique of the worker W and of a naturally determined location in front of the worker W which is separated from the worker W by a distance in accordance with the assumed physique. The ratio of the area (or volume) of a region included in the front working region in the working region with respect to the area (or volume) of the working region in which the worker W actually performs work is referred to as "front performance factor". According to the robot device equipped with the linear extension and retraction arm mechanism of the present embodiment, it is possible to arrange the robot device in the vicinity of the worker W, and by this means the work of the worker W is assisted by the robot device and thus a layout can be adopted in which the working region of the worker W is made as small as possible and is set at the front of the worker W, thereby enabling an improvement in the front performance factor. That is, by the robot device of the present embodiment assisting the work of the worker W, the worker W can perform almost all work within the front working region, thereby improving the working efficiency.

A feature according to the present embodiment is that the base 1 of the robot device can be disposed in the vicinity of the worker W, and this feature is clarified by a description regarding the distance between the base 1 of the robot device and the worker W. In this case, although the shortest distance between the center of the base 1 of the robot device and the outer circumference of the occupied region OR of the worker W is described as a definition of the distance between the base 1 of the robot device and the worker W, the definition of the distance between the base 1 of the robot device and the worker W is not limited thereto, and for example the aforementioned distance may be defined as the distance from the center of the base 1 of the robot device to the center of the occupied region OR of the worker W, or may be defined as the distance of an interval between the base 1 of the robot device and the occupied region OR of the worker W. These definitions can be said to be substantially the same definition, and a distance from the center of the base 1 of the robot device to the outer circumference can be easily converted using the radius of the occupied region OR of the worker W.

The base 1 of the robot device can be laid out at a position at which the center thereof is separated from the outer circumference of the occupied region OR of the worker W by a distance Dne that is shorter than the length RL1 (long) from the first axis RA1 to the tip of the arm section 2 when the arm section 2 is extended to the maximum extent. In other words, the base 1 of the robot device can be laid out within the length RL1 (long) from the worker W.

Furthermore, as shown in FIG. 7B, it is also possible to lay out the base 1 of the robot device adjacent to the worker W so that the center of the base 1 is at a position that is separated from the outer circumference of the occupied region OR of the worker W by a distance Dne that is shorter than a length RL1 (short) from the first axis RA1 to the tip of the arm section 2 when the arm section 2 is retracted to the maximum extent.

According to this layout, the reach region MM of the worker W overlaps with the moveable region MR(L) of the arm section 2. Further, the moveable region MR(L) of the arm section 2 overlaps with the working region of the worker W. In addition, within the moveable region MR(L) of the arm section 2, the working region assigned to the robot device overlaps with the working region assigned to the worker W. In the example in FIG. 7A, the moveable region MR(L) of the arm section 2 partially overlaps with the front working region of the worker W, and in the example in FIG. 7B, the moveable region MR(L) of the arm section 2 includes the front working region of the worker W.

Note that, at the time of actual operations, the robot device is subjected to teaching in accordance with the working region WAR and the content of work that is assigned to the robot device as well as the locations of interference objects such as the worker W around the robot device. A region over which the arm section 2 sweeps in accordance with an operating range (operational turning angle) around the first axis RA1 of the first joint J1, an operating range (operational upward/downward rotation angle) around the second axis RA2 of the second joint J2 and an operating range (operational extension length) of the third joint J3 as well as operating ranges (operational rotation angles) of the joints J4, J5 and J6 that are the wrist three axes that are set by the aforementioned teaching is referred to as an "operating region". Naturally, the operating region is included in the moveable region MR, and is distinguished from the moveable region MR.

The above described layout can be realized, because the linear extension and retraction arm mechanism of the robot device according to the present embodiment does not have an elbow joint, the joints or the like substantially do not project to the outside of the substantially fan-shaped moveable region MR, the worker W is positioned at the same side as that of a turning center RA1 of the arm section 2, the turning trajectory of the arm section 2 in which there is a high possibility of contact with the worker W can be predicted with a high degree of predictability based on the motion of the end, and hence danger caused by the arm section 2 can be further reduced.

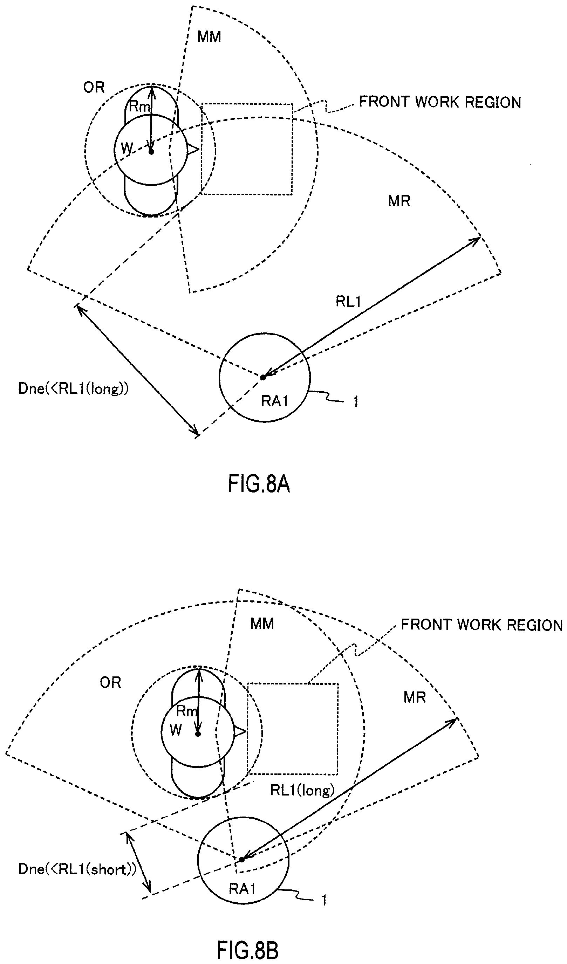

FIG. 8A shows an example of a layout in which the base 1 of the robot device is disposed in front of the worker W at a position that is at an angle of approximately 90 degrees relative to the worker W. The base 1 of the robot device can be laid out at a position at which the center thereof is separated from the outer circumference of the occupied region OR the center of which is a planned position (center position of the occupied region OR of the worker W) at which the worker W is to stand during the work by a distance Dne that is shorter than the length RL1 (long) from the first axis RA1 to the tip of the arm section 2 when the arm section 2 is extended to the maximum extend. In addition, as shown in FIG. 8B, it is also possible to lay out the base 1 of the robot device adjacent to the worker W so that the center of the base 1 is at a position that is separated from the outer circumference of the occupied region OR of the worker W by a distance Dne that is shorter than the length RL1 (short) from the first axis RA1 to the tip of the arm section 2 when the arm section 2 is retracted to the maximum extent.

By setting the distance Dne to be shorter than the length RL1 (long), and also to be shorter than the length RL1 (short), the moveable region MR of the arm mechanism 200 interferes with the occupied region OR of the worker W. However, the operating region of the arm section 2 is set by teaching so as to include the working region WAR assigned to the robot device and not to interfere with the occupied region OR of the worker W. The relevant distance Dne between the base 1 and the worker W makes it possible for the moveable region MR and the reach region MM to partially overlap and, in addition, can cause the working region WAR assigned to the robot device within the moveable region MR to partially overlap with the working region WAM inside the reach region MM of the worker W as shown in FIG. 11, and such a sufficiently wide overlapping region allows the robot device to perform work in collaboration with the worker on the same work piece.

In the example in FIG. 8A, the moveable region MR(L) of the arm section 2 partially overlaps with the front working region of the worker W, and in the example in FIG. 8B, it is possible for the moveable region MR(L) of the arm section 2 to include the front working region of the worker W, and thus the working region of the worker can be designed to allow the robot device to assist the work of the worker W and improve the front performance factor.

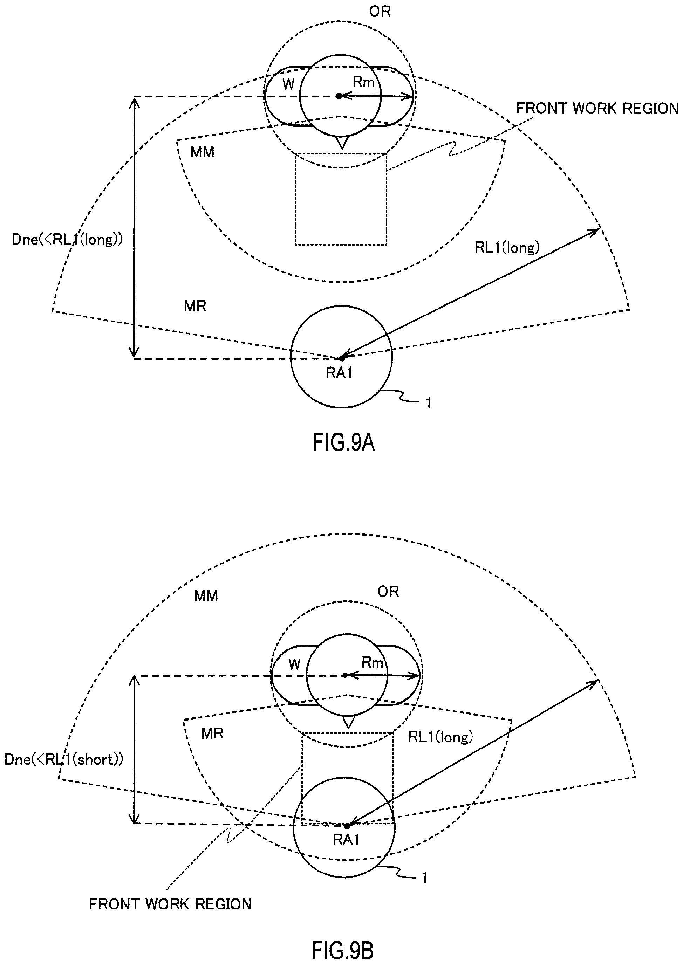

FIG. 9A illustrates an example of a layout in which the base 1 of the robot device is disposed in a direction facing the worker W. The base 1 of the robot device can be laid out at a position at which the center thereof is separated from the outer circumference of the occupied region OR the center of which is a planned position (center position of the occupied region OR of the worker W) at which the worker W is to stand during the work by the distance Dne that is shorter than the length RL1 (long) from the first axis RA1 to the tip of the arm section 2 when the arm section 2 is extended to the maximum extent. In addition, as shown in FIG. 9B, it is possible to lay out the base 1 of the robot device adjacent to the worker W so that the center of the base 1 is at a position that is separated from the outer circumference of the occupied region OR of the worker W by a distance Dne that is shorter than the length RL1 (short) from the first axis RA1 to the tip of the arm section 2 when the arm section 2 is retracted to the maximum extent.

By setting the distance Dne to be shorter than the length RL1 (long), and also to be shorter than the length RL1 (short), the moveable region MR of the arm mechanism 200 interferes with the occupied region OR of the worker W. However, the operating region of the arm mechanism 200 is set by teaching so as to include the working region WAR that is assigned to the robot device and not to interfere with the occupied region OR of the worker W. The relevant distance Dne between the base 1 and the worker W allows the moveable region MR to include the reach region MM and can cause the working region WAR that is assigned to the robot device within the moveable region MR to partially overlap with the working region WAM inside the reach region MM of the worker W.

In the examples shown in FIG. 9A and FIG. 9B, it is possible for the moveable region MR(L) of the arm section 2 to include the front working region of the worker W, and by this means the working region of the worker can be designed to allow the robot device to assist the work of the worker W and improve the front performance factor.

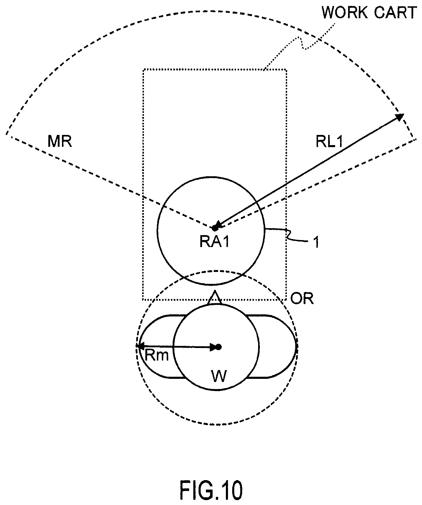

FIG. 10 shows an example of a layout in which the base 1 of the robot device is disposed directly in front of the worker W and facing in the same direction as that of the worker W. The base 1 of the robot device may be laid out at a position that is directly in front of the planned position at which the worker W is to stand during the work, and the base 1 of the robot device may also be laid out at a position that is inside the occupied region OR of the worker W. In this case, for example, a teaching specialist is supposed as the worker W, or a situation is supposed in which the base 1 of the robot device is mounted on a work cart and, for example, the robot device and the worker W who pushes the work cart collaborate to gather required parts from parts shelves on the left and right of a parts warehouse.

In the linear extension and retraction arm mechanism of the robot device according to the present embodiment, because there is no elbow joint and the joints or the like do not project to the outside of the substantially fan-shaped region MR, by the worker W standing at the back face of the base 1 of the robot device and performing teaching operations, the danger of the end, the arm section 2 and any of the joints J1 to J6 contacting with the worker W is eliminated, and furthermore the worker W can intuitively understand the reference coordinate system ∑b of the robot device at a position that is close to the base 1 thereof and can efficiently execute teaching operations.

As shown in FIG. 11, work with respect to a work piece (work) is assigned to the robot device within the working region WAR. The working region WAR is defined at an initial stage of the workspace design. In the workspace design, in order to allow the robot device to perform work in collaboration with the worker W on the work piece that is on a work bench inside the working region WAR, the layout of the substantially fan-shaped region MR and a central angle θ are designed (setting of the operating region) so as to include the working region WAR and to also avoid interference objects such as a worker, a tool shelf and a wall surface in the area around the robot device, and the position of the base 1 is determined as the fan center of the substantially fan-shaped region MR that is obtained as a result.

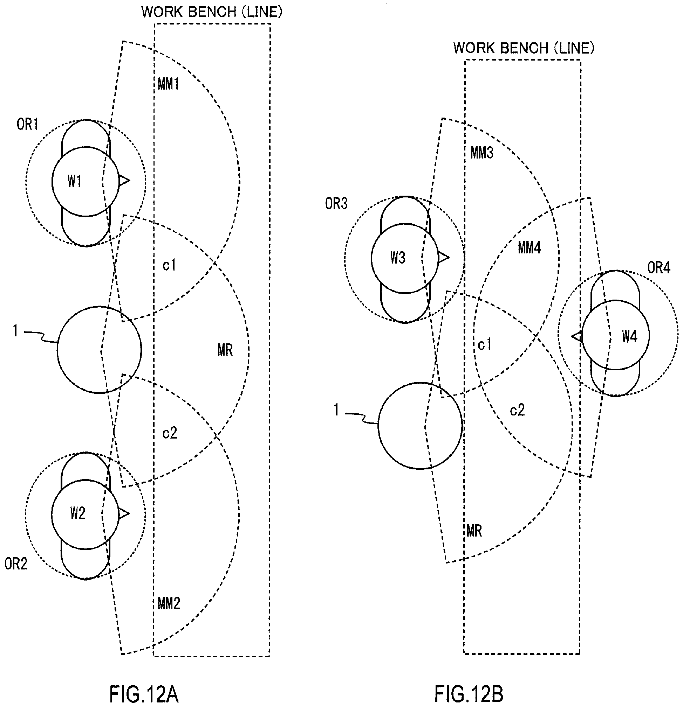

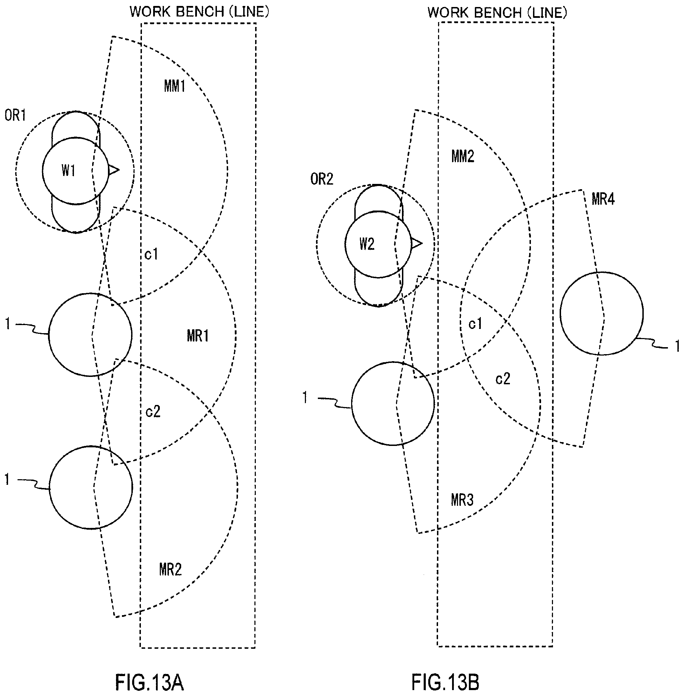

A plurality of the layouts described above can be arbitrarily combined and practically expanded as shown in FIGS. 12A and 12B and FIGS. 13A and 13B. As shown in FIG. 12A, a layout can be adopted in which the base 1 of the robot device and two workers W1 and W2 are positioned along a work bench, for example, an assembly line, with the base 1 of the robot device located between the two workers W1 and W2, and with the two workers W1 and W2 separated from the robot device by a distance that is shorter than the length RL1 (long) from the first axis RA1 to the tip of the arm section 2 when the arm section 2 is extended to the maximum extent, and furthermore, depending on the circumstances, shorter than the length RL1 (short). According to this layout, in a region C1 the moveable region MR partially overlaps with a reach region MM1 of one worker W1, and also overlaps in a region C2 with a reach region MM2 of the other worker W2. This layout makes it possible for a single robot device to perform work in collaboration with two workers W.

As shown in FIG. 12B, the base 1 of the robot device and a worker W3 are positioned on one side of a work bench, for example, an assembly line, and a worker W4 is positioned on the opposite side of the line. A layout can be adopted in which the base 1 of the robot device is at a position that is adjacent to one of the workers, i.e. the worker W3, and that approximately faces the other worker W4. The base 1 of the robot device can be laid out at a position that is separated from the adjacent worker W3 by a distance that is shorter than the length RL1 (long) from the first axis RA1 to the tip of the arm section 2 when the arm section 2 is extended to the maximum extent, and a position that is separated from the facing other worker W4 by the distance Dne that is shorter than the length RL1 (long) from the first axis RA1 to the tip of the arm section 2 when the arm section 2 is extended to the maximum extent. According to this layout, the moveable region MR partially overlaps with the reach region MM3 of the adjacent worker W3 in the region C1, and overlaps in the region C2 with the reach region MM2 of the worker W4 who is positioned in front of the robot device. In addition, this layout also enables a single robot device to perform work in collaboration with two workers W.

As shown in FIG. 13A, a layout can be adopted in which the bases 1 of two robot devices R1 and R2 are adjacent to a worker W1 in a single line along a work bench, for example, an assembly line. The base 1 of one of the robot devices, i.e. the robot device R1, can be laid out at a position that is separated by a distance that is shorter than the length RL1 (long) from the worker W1, and the base 1 of the other robot device R2 can be laid out at a position that is separated by a distance that, for example, is shorter than the length RL1 (long) from the base 1 of one robot device R1. According to this layout, a moveable region MR1 of one robot device R1 partially overlaps in a region C1 with the reach region MM1 of the worker W1, and moveable regions MR1 and MR2 of the two robot devices R1 and R2 overlap with each other in a region C2. This layout makes it possible for two robot devices to collaborate with the worker W to perform work.

Similarly, as shown in FIG. 13B, the base 1 of one robot device R3 can be laid out adjacent to a worker W2 along one side of a work bench, for example, an assembly line, and the base 1 of the other robot device R4 can be laid out at a position that approximately faces the worker W2 on the opposite side of the line. The base 1 of one of the robot devices, i.e. the robot device R3, is laid out at a position that is separated from the worker W2 by a distance that is shorter than the length RL1 (long), and the base 1 of the other robot device R4 is laid out at a position that is separated from the worker W2 by a distance that is shorter than the length RL1 (long). According to this layout, a moveable region MR3 of the robot device R3 partially overlaps in a region C1 with a reach region MM2 of the worker W2, and a moveable region MR4 of the robot device R4 also overlaps at least partially with the reach region MM2 of the worker W2 in a region C2. In addition, the moveable region MR3 of the robot device R3 and the moveable region MR4 of the robot device R4 overlap with each other. This layout makes it possible for two robot devices to perform work in collaboration with the worker W, and also makes it possible for two robot devices to perform work in collaboration with each other.

While certain embodiments have been described, these embodiments have been presented by way of example only, and are not intended to limit the scope of the inventions. Indeed, the novel methods and systems described herein may be embodied in a variety of other forms; furthermore, various omissions, substitutions and changes in the form of the methods and systems described herein may be made without departing from the spirit of the inventions. The accompanying claims and their equivalents are intended to cover such forms or modifications as would fall within the scope and spirit of the inventions.

### Reference Signs List

1...base, 2...arm section, 3...end effector, J1, J2, J4, J5, J6...rotary joint, J3...linear motion joint, 11a...first support body, 11b...second support body, 11c...third support body, 20...second connection piece string, 21...first connection piece string, 22...second connection piece, 23...first connection piece, 26...joining piece, MR...moveable region of robot device, MM...reach region of worker, WAR...work region of robot device, WAM...work region of worker, W...worker, OR...occupied region.

## Claims

1. A robot system, comprising:
a work bench on which a work piece is placed; and
a robot device which comprises a base, and an arm mechanism having torsional rotatability around a first axis approximately along a center line of the base, bending rotatability around a second axis that is orthogonal to the first axis, and linear extensibility and retractability along a third axis that is orthogonal to the second axis, and which is used for performing work in collaboration with a worker with respect to the work piece by means of an end effector that is mounted at a tip of the arm mechanism;
wherein the base is disposed so that a moveable region of the arm section accompanying rotation around the first axis and the second axis and linear extension and retraction along the third axis overlaps a working region of the worker.

2. The robot system according to claim 1, wherein
the base is disposed at a position that is adjacent to the worker as viewed from the work bench.

3. The robot system according to claim 1, wherein
the base is disposed at a position that faces the worker in a manner that interposes the work bench between the base and the worker.

4. A robot system, comprising:
a work bench on which a work piece is placed; and
a robot device which comprises a base, and an arm section having torsional rotatability around a first axis approximately along a center line of the base, bending rotatability around a second axis that is orthogonal to the first axis, and linear extensibility and retractability along a third axis that is orthogonal to the second axis, and which is used for performing work in collaboration with a worker with respect to the work piece by means of an end effector that is mounted at a tip of the arm section;
wherein the base is disposed so that a working region assigned to the robot device in a moveable region of the arm section accompanying rotation around the first axis and the second axis and linear extension and retraction along the third axis overlaps with a working region of the worker.

5. A robot system, comprising:
a work bench on which a work piece is placed; and
a robot device which comprises a base, and an arm section having torsional rotatability around a first axis approximately along a center line of the base, bending rotatability around a second axis that is orthogonal to the first axis, and linear extensibility and retractability along a third axis that is orthogonal to the second axis, and which is used for performing work in collaboration with a worker with respect to the work piece by means of an end effector that is mounted at a tip of the arm section;
wherein the base is disposed so that a moveable region of the arm section accompanying rotation around the first axis and the second axis and linear extension and retraction along the third axis interferes with the worker.

6. A robot system, comprising:
a work bench on which a work piece is placed; and
a robot device which comprises a base, and an arm section having torsional rotatability around a first axis approximately along a center line of the base, bending rotatability around a second axis that is orthogonal to the first axis, and linear extensibility and retractability along a third axis that is orthogonal to the second axis, and which is used for performing work with respect to the work piece by means of an end effector that is mounted at a tip of the arm section;
wherein the base is disposed so that a worker is positioned in a vicinity of a back side of the base that is a position that is outside a moveable region of the arm section accompanying rotation around the first axis and the second axis and linear extension and retraction along the third axis.

7. A robot system, comprising:
a work bench on which a work piece is placed; and
a robot device which comprises a base, and an arm section having torsional rotatability around a first axis approximately along a center line of the base, bending rotatability around a second axis that is orthogonal to the first axis, and linear extensibility and retractability along a third axis that is orthogonal to the second axis, and which is used for performing work with respect to the work piece by means of an end effector that is mounted at a tip of the arm section;
wherein the base is disposed so that a worker is positioned inside a substantially fan-shaped region the center of which is the first axis and which takes as a radius a length from the first axis to the tip of the arm section when the arm section is extended maximum.

8. A robot system, comprising:
a work bench on which a work piece is placed; and
a plurality of robot devices which each comprise a base, and an arm section having torsional rotatability around a first axis approximately along a center line of the base, bending rotatability around a second axis that is orthogonal to the first axis, and linear extensibility and retractability along a third axis that is orthogonal to the second axis, and which are used for performing work with respect to the work piece by means of an end effector mounted at a tip of the arm section;
wherein each of the bases of the plurality of robot devices is disposed so that moveable regions of the arm sections accompanying rotation around the first axis and the second axis and linear extension and retraction along the third axis overlap with each other among the plurality of robot devices.

9. A robot system, comprising:
a work bench on which a work piece is placed; and
a robot device which comprises a base, and an arm section having torsional rotatability around a first axis approximately along a center line of the base, bending rotatability around a second axis that is orthogonal to the first axis, and linear extensibility and retractability along a third axis that is orthogonal to the second axis, and which is installed in a vicinity of the work bench and is used for performing work in collaboration with a worker with respect to the work piece by means of an end effector that is mounted at a tip of the arm section;
wherein the base is disposed at a position that is at a distance to the worker shorter than a length from the first axis to the tip of the arm section when the arm section is extended maximum.

10. A robot system, comprising:
a work bench on which a work piece is placed; and
a robot device which comprises a base, and an arm section having torsional rotatability around a first axis approximately along a center line of the base, bending rotatability around a second axis that is orthogonal to the first axis, and linear extensibility and retractability along a third axis that is orthogonal to the second axis, and which is installed in a vicinity of the work bench and is used for performing work in collaboration with a worker with respect to the work piece by means of an end effector that is mounted at a tip of the arm section;
wherein the base is disposed at a position that is at a distance to the worker shorter than a length from the first axis to the tip of the arm section when the arm section is retracted to a maximum extent.

11. A robot system, comprising:
a work bench on which a work piece is placed; and
a robot device which comprises a base, and an arm section having torsional rotatability around a first axis approximately along a center line of the base, bending rotatability around a second axis that is orthogonal to the first axis, and linear extensibility and retractability along a third axis that is orthogonal to the second axis, and which is installed in a vicinity of the work bench and is used for performing work with respect to the work piece by means of an end effector that is mounted at a tip of the arm section;
wherein a substantially fan-shaped region that takes a length from the first axis to the tip of the arm section as a radius is arranged so as to include a working region assigned to the robot device, and the base is disposed at a fan center of the substantially fan-shaped region.

12. A robot system, comprising:
a work bench on which a work piece is placed; and
a robot device which comprises a base, and an arm section having torsional rotatability around a first axis approximately along a center line of the base, bending rotatability around a second axis that is orthogonal to the first axis, and linear extensibility and retractability along a third axis that is orthogonal to the second axis, and which is installed in a vicinity of the work bench and is used for performing work with respect to the work piece by means of an end effector that is mounted at a tip of the arm section;
wherein a substantially fan-shaped region that takes a length from the first axis to the tip of the arm section as a radius is arranged so as to interfere with an interference object in a vicinity thereof, and the base is disposed at a fan center of the substantially fan-shaped region.

13. A robot system, comprising:
a work bench on which a work piece is placed; and
a robot device which comprises a base, and an arm section having torsional rotatability around a first axis approximately along a center line of the base, bending rotatability around a second axis that is orthogonal to the first axis, and linear extensibility and retractability along a third axis that is orthogonal to the second axis, and which is used for performing work in collaboration with a worker with respect to the work piece by means of an end effector that is mounted at a tip of the arm section;
wherein the base is disposed at a position at which a moveable region of the arm section accompanying rotation around the first axis and the second axis and linear extension and retraction along the third axis overlaps with an assumed front working region in front of the worker.

14. A robot device, comprising:
a base, and
an arm section having torsional rotatability around a first axis approximately along a center line of the base, bending rotatability around a second axis that is orthogonal to the first axis, and linear extensibility and retractability along a third axis that is orthogonal to the second axis,
the robot device being used for performing work in collaboration with a worker with respect to a work piece by means of an end effector that is mounted at a tip of the arm section;
wherein the base is disposed at a position at which a moveable region of the arm section accompanying rotation around the first axis and the second axis and linear extension and retraction along the third axis overlaps with a working region of the worker.

15. The robot device according to claim 14, wherein the base is disposed at a position that is adjacent to the worker.

16. The robot device according to claim 14, wherein the base is disposed at a position that faces the worker.

17. A robot device, comprising:
a base, and
an arm section having torsional rotatability around a first axis approximately along a center line of the base, bending rotatability around a second axis that is orthogonal to the first axis, and linear extensibility and retractability along a third axis that is orthogonal to the second axis,
the robot device being used for performing work in collaboration with a worker with respect to a work piece by means of an end effector that is mounted at a tip of the arm section;
wherein the base is disposed at a position at which a working region assigned to the robot device in a moveable region of the arm section accompanying rotation around the first axis and the second axis and linear extension and retraction along the third axis overlaps with a working region of the worker.

18. A robot device, comprising:
a base, and
an arm section having torsional rotatability around a first axis approximately along a center line of the base, bending rotatability around a second axis that is orthogonal to the first axis, and linear extensibility and retractability along a third axis that is orthogonal to the second axis,
the robot device being used for performing work in collaboration with a worker with respect to a work piece by means of an end effector that is mounted at a tip of the arm section;
wherein the base is disposed so that a moveable region of the arm section accompanying rotation around the first axis and the second axis and linear extension and retraction along the third axis interferes with a working region of the worker.

19. A robot device, comprising:
a base, and
an arm section having torsional rotatability around a first axis approximately along a center line of the base, bending rotatability around a second axis that is orthogonal to the first axis, and linear extensibility and retractability along a third axis that is orthogonal to the second axis,
the robot device being used for performing work with respect to a work piece by means of an end effector that is mounted at a tip of the arm section;
wherein the base is disposed so that a worker is positioned in a vicinity of a back side of the base that is a position that is outside a moveable region of the arm section accompanying rotation around the first axis and the second axis and linear extension and retraction along the third axis.

20. A robot device, comprising:
a base, and
an arm section having torsional rotatability around a first axis approximately along a center line of the base, bending rotatability around a second axis that is orthogonal to the first axis, and linear extensibility and retractability along a third axis that is orthogonal to the second axis,
the robot device being used for performing work with respect to a work piece by means of an end effector that is mounted at a tip of the arm section;
wherein the base is disposed so that a worker is positioned inside a substantially fan-shaped region the center of which is the first axis and which takes as a radius a length from the first axis to the tip of the arm section when the arm section is extended to a maximum extent.

21. A robot device, comprising:
a base, and
an arm section having torsional rotatability around a first axis approximately along a center line of the base, bending rotatability around a second axis that is orthogonal to the first axis, and linear extensibility and retractability along a third axis that is orthogonal to the second axis,
the robot device being used for performing work with respect to a work piece by means of an end effector that is mounted at a tip of the arm section;
wherein the base is disposed so that a moveable region of the arm section accompanying rotation around the first axis and the second axis and linear extension and retraction along the third axis overlaps in a mutual manner with a moveable region of another robot device.

22. A robot device, comprising:
a base, and
an arm section having torsional rotatability around a first axis approximately along a center line of the base, bending rotatability around a second axis that is orthogonal to the first axis, and linear extensibility and retractability along a third axis that is orthogonal to the second axis,
the robot device being installed in a vicinity of a work bench and being used for performing work in collaboration with a worker with respect to a work piece by means of an end effector that is mounted at a tip of the arm section;
wherein the base is disposed at a position that is at a distance to the worker shorter than a length from the first axis to the tip of the arm section when the arm section is extended to a maximum extent.

23. A robot device, comprising:
a base, and
an arm section having torsional rotatability around a first axis approximately along a center line of the base, bending rotatability around a second axis that is orthogonal to the first axis, and linear extensibility and retractability along a third axis that is orthogonal to the second axis,
the robot device being installed in a vicinity of a work bench and being used for performing work in collaboration with a worker with respect to a work piece by means of an end effector that is mounted at a tip of the arm section;
wherein the base is disposed at a position that is at a distance to the worker shorter than a length from the first axis to the tip of the arm section when the arm section is retracted to a maximum extent.

24. A robot device, comprising:
a base, and
an arm section having torsional rotatability around a first axis approximately along a center line of the base, bending rotatability around a second axis that is orthogonal to the first axis, and linear extensibility and retractability along a third axis that is orthogonal to the second axis,
the robot device being installed in a vicinity of a work bench and being used for performing work with respect to a work piece by means of an end effector that is mounted at a tip of the arm section;
wherein a substantially fan-shaped region that takes a length from the first axis to the tip of the arm section as a radius is arranged so as to include a working region assigned to the robot device, and the base is disposed at a fan center of the substantially fan-shaped region.

25. A robot device, comprising:
a base, and
an arm section having torsional rotatability around a first axis approximately along a center line of the base, bending rotatability around a second axis that is orthogonal to the first axis, and linear extensibility and retractability along a third axis that is orthogonal to the second axis,
the robot device being installed in a vicinity of a work bench and being used for performing work with respect to a work piece by means of an end effector that is mounted at a tip of the arm section;
wherein a substantially fan-shaped region that takes a length from the first axis to the tip of the arm section as a radius is arranged so as to interfere with an interference object in a vicinity of the robot device, and the base is disposed at a fan center of the substantially fan-shaped region.

26. A robot device, comprising:
a base, and
an arm section having torsional rotatability around a first axis approximately along a center line of the base, bending rotatability around a second axis that is orthogonal to the first axis, and linear extensibility and retractability along a third axis that is orthogonal to the second axis,
the robot device being installed in a vicinity of a work bench and being used for performing work with respect to a work piece by means of an end effector that is mounted at a tip of the arm section;
wherein the base is disposed at a position at which a moveable region of the arm section accompanying rotation around the first axis and the second axis and linear extension and retraction along the third axis overlaps with an assumed front working region in front of a worker.
